Europäisches Patentamt

European Patent Office

Office européen des brevets

(11) Numéro de publication : **0 439 394 B1**

(12) **FASCICULE DE BREVET EUROPEEN**

(45) Date de publication du fascicule du brevet :
30.03.94 Bulletin 94/13

(51) Int. Cl.⁵ : **B29D 11/00**, G02C 7/02

(21) Numéro de dépôt : **91400131.8**

(22) Date de dépôt : **21.01.91**

(54) Procédé pour la fabrication d'une lentille optique artificielle, et lentille optique artificielle correspondante.

(30) Priorité : **22.01.90 FR 9000679**
**16.11.90 FR 9014282**

(43) Date de publication de la demande :
**31.07.91 Bulletin 91/31**

(45) Mention de la délivrance du brevet :
**30.03.94 Bulletin 94/13**

(84) Etats contractants désignés :
**BE CH DE ES GB IT LI NL SE**

(56) Documents cités :
**EP-A- 0 145 392**
**EP-A- 0 207 640**
**FR-A- 2 029 178**
**FR-A- 2 076 194**
**GB-A- 618 787**
**GB-A- 636 283**
**GB-A- 636 379**
**US-A- 3 993 485**
**US-A- 4 541 969**
**PATENT ABSTRACTS OF JAPAN, vol. 10, no. 216 (M-502)[2272], 29 juillet 1986; & JP-A-61 053 031 (MATSUSHITA ELECTRIC WORKS LTD) 15-03-1986**
**PATENT ABSTRACTS OF JAPAN, vol. 8, no. 180 (M-318)[1617], 18 août 1984; & JP-A-59 071 830 (NIHON ITA GLASS K.K.) 23-04-1984**

(73) Titulaire : **ESSILOR INTERNATIONAL, Cie Générale d'Optique**
**1 Rue Thomas Edison, Echat 902**
**F-94028 Créteil Cédex (FR)**

(72) Inventeur : **Baude, Dominique**
**1 Allée du 8 Mai 1945**
**F-93400 Saint Ouen (FR)**
Inventeur : **Meslard, Jean-Claude**
**45 Rue du Maréchal Leclerc**
**F-94410 Saint Maurice (FR)**
Inventeur : **Obrecht, Gérard**
**20 Rue Emile Zola**
**F-92130 Issy Les Moulineaux (FR)**
Inventeur : **Chavel, Pierre**
**6 Avenue de Rocroi, Le Bois des Ormes**
**F-91380 Chilly-Mazarin (FR)**
Inventeur : **Joyeux, Denis**
**51 Rue des Chardonnerets**
**F-91840 Les Ulis (FR)**
Inventeur : **Taboury, Jean**
**89 Rue Houdan**
**F-92330 Sceaux (FR)**

(74) Mandataire : **CABINET BONNET-THIRION**
**95 Boulevard Beaumarchais**
**F-75003 Paris (FR)**

EP 0 439 394 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne d'une manière générale les lentilles optiques artificielles, c'est-à-dire aussi bien les lentilles de contact ou les implants intra-oculaires que les lentilles ophtalmiques, c'est-à-dire les verres de lunettes, et elle vise plus particulièrement celles de ces lentilles optiques artificielles qui, pour assurer une correction, doivent présenter radialement un quelconque profil de puissance déterminé.

La fabrication, par exemple, de lentilles de contact se fait actuellement soit par moulage, soit par usinage.

La fabrication par moulage a pour avantage de pouvoir conduire directement au profil de puissance recherché, par simple duplication à partir d'un moule approprié.

Mais, en pratique, seul peut alors être modulé le rayon de coubure externe des lentilles de contact artificielles fabriquées, leur rayon de courbure interne devant rester dans des limites étroites et bien déterminées, pour une bonne adaptation au rayon de courbure de la cornée des patients.

Il en résulte qu'une fabrication par moulage impose de disposer d'un parc de moules important.

Ce parc de moules doit en effet pouvoir permettre de couvrir toutes les gammes de puissances nécessaires à la correction de toutes les amétropies.

Pour les lentilles de contact pour presbytes, ce parc de moules doit en outre pouvoir permettre de couvrir toutes les gammes d'additions de puissance nécessaires à la correction de toutes les presbyties et ce pour chaque amétropie à corriger.

Une fabrication par usinage, qui consiste à reprendre, par un processus de traitement mécanique, jusqu'à lui conférer le profil de puissance recherché, un produit semi-fini préalablement obtenu par moulage, permet avantageusement de simplifier le parc de moules nécessaire.

Mais, en pratique, les processus de traitement alors à mettre en oeuvre sont lourds, délicats et onéreux.

Il a par ailleurs déjà été proposé d'appliquer à des lentilles optiques un processus de traitement physico-chimique.

C'est le cas, par exemple, dans les brevets français publiés sous les Nos 2.029.178 et 2.076.194.

Mais, ces brevets français concernent essentiellement l'obtention de verres correcteurs destinés à l'équipement de montures de lunettes, et si, dans le premier, il est fait référence à l'irradiation possible de lentilles de contact, cette irradiation, qui est en pratique une irradiation neutronique, n'y a pour but que de supprimer certains défauts de ces lentilles de contact.

Il ne s'agit donc pas, dans ces brevets français, d'assurer la fabrication de lentilles optiques artificielles présentant un quelconque profil de puissance donné.

Si, par ailleurs, il est proposé, dans le document de brevet japonais No JP-A-61 053031, la mise en oeuvre d'un traitement physico-chimique pour l'obtention d'un certain profil de puissance, avec, pour la modulation spatiale du flux d'énergie à contrôler, l'intervention d'un masque, le substrat de départ n'est qu'une simple lame à faces parallèles.

En outre, le masque mis en oeuvre est un masque statique, qui intervient en quelque sorte par niveaux de gris, et dont il est difficile en fait de contrôler le noircissement.

La présente invention a notamment pour objet un procédé qui, propre à permettre la fabrication d'une lentille optique artificielle présentant un quelconque profil de puissance donné, est avantageusement exempt de ces inconvénients.

Ce procédé est du genre suivant lequel, partant d'une lentille optique artificielle présentant un autre profil de puissance, on assure par un processus de traitement physico-chimique, une modification de ce profil de puissance conduisant au profil de puissance recherché, avec, pour la modulation spatiale du flux d'énergie à contrôler, l'intervention d'un masque, et il est d'une manière générale caractérisé en ce que on met en oeuvre, pour la modulation spatiale du flux d'énergie, un masque tournant.

Ainsi, comme pour une fabrication par usinage, il est procédé, suivant l'invention, à une reprise d'un produit semi-fini de départ, qui, de ce fait, peut avantageusement être plus simple, et, par exemple, être une simple lentille sphérique.

Mais, suivant l'invention, le processus de reprise correspondant est avantageusement un processus de traitement physico-chimique.

Il s'avère en effet que, soit par une modification locale de l'indice de réfraction du matériau constitutif de la lentille traitée, soit par une modification locale de son épaisseur, soit par une modification portant à la fois sur cet indice de réfraction et sur cette épaisseur, les processus de traitement physico-chimique accessibles à ce jour peuvent effectivement permettre une modification du profil de puissance d'une telle lentille, c'est-à-dire une modification de ses caractéristiques optiques la conduisant à présenter le profil de puissance recherché.

Les processus de traitement physico-chimique ainsi susceptibles d'être mis en oeuvre sont très divers.

Il peut s'agir par exemple aussi bien de photochimie que de dopage d'ions, de photoablation ou d'usinage ionique.

Quoi qu'il en soit, le fait que, suivant l'invention, la modulation spatiale du flux d'énergie soit assurée à l'aide d'un masque tournant permet un meilleur contrôle de cette modulation.

Il s'avère, en effet, que la précision suivant laquelle le tracé d'un tel masque tournant est susceptible d'être réalisé est largement supérieure à celle suivant laquelle il est actuellement possible de contrôler le noircissement d'un masque statique à niveaux de gris.

Suivant un développement de l'invention, le masque tournant ainsi mis en oeuvre se prête en outre avantageusement à l'obtention d'un profil de puissance astigmate si désiré.

Il s'avère, en effet, que, de manière relativement simple, il suffit de moduler la vitesse de rotation du masque tournant suivant sa position angulaire relative par rapport à la lentille optique artificielle en cours de traitement pour obtenir un profil de puissance astigmate pour celle-ci.

La présente invention a encore pour objet toute lentille optique artificielle obtenue en application du procédé précédemment succinctement exposé, c'est-à-dire toute lentille optique artificielle présentant des caractéristiques optiques résultant d'une modification par processus physico-chimique de caractéristiques optiques initialement différentes.

Les objets de l'invention, leurs caractéristiques et leurs avantages, ressortiront d'ailleurs de la description qui va suivre, à titre d'exemple, en référence aux dessins schématiques annexés sur lesquels :

la figure 1 est une vue partielle en coupe axiale d'une lentille optique artificielle suivant l'invention ;

les figures 2 et 3 sont deux blocs diagrammes s'y rapportant, ces deux blocs diagrammes illustrant, l'un, la variation de puissance de la lentille en fonction de la distance à l'axe optique, l'autre la variation de phase correspondante ;

la figure 4 est une vue en plan d'un masque tournant susceptible d'être mis en oeuvre pour sa fabrication ;

les figures 5, 6 sont des vues respectivement analogues à celles des figures 3, 4, pour une variante de réalisation ;

les figures 7, 8, 9 sont des vues respectivement analogues à celles des figures 2, 3, 4, pour une autre lentille optique artificielle suivant l'invention ;

les figures 10A, 10B sont des blocs diagrammes qui, analogues à celui de la figure 5, se rapportent à cette autre lentille optique artificielle ;

la figure 11 est, pour elle, une vue analogue à celle de la figure 6 ;

la figure 12 est une vue en plan de la zone optique utile d'une lentille optique artificielle à laquelle est susceptible d'être appliqué un développement de l'invention ;

la figure 13 est un diagramme de nature à faciliter l'exposé concernant ce développement ;

la figure 14 est une vue en plan d'un masque tournant s'y rapportant ;

la figure 15 est un diagramme relatif à l'un des paramètres dont dépend la réalisation de ce masque tournant.

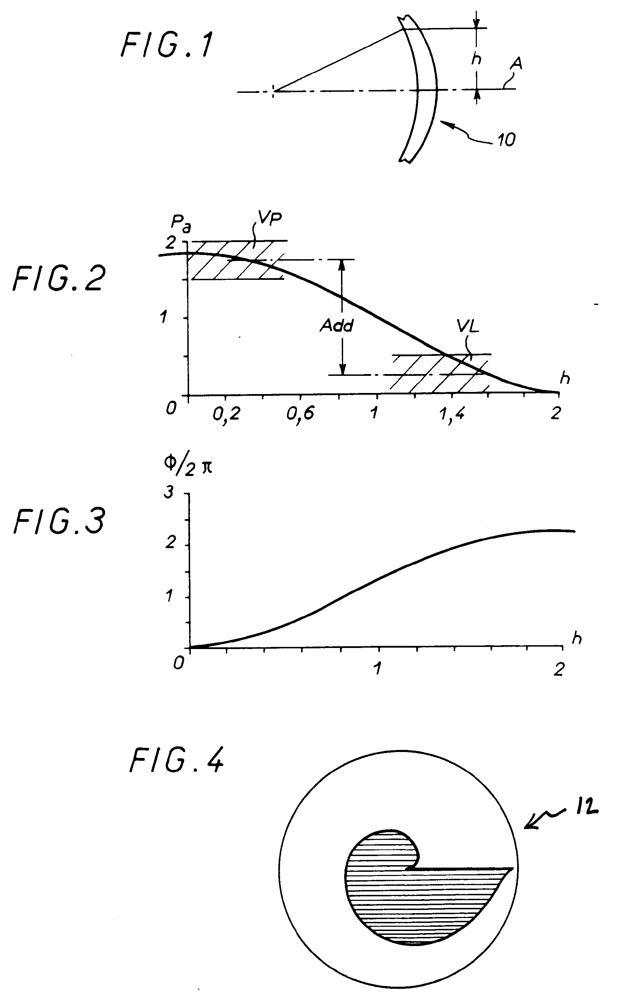

Tel qu'illustré à la figure 1, il s'agit par exemple de la fabrication d'une lentille de contact 10, et, plus précisément, d'une lentille de contact 10 à vision simultanée, qui, pour la correction d'une presbytie, a une puissance P(h) variant radialement suivant un profil asphérique déterminé.

Cette puissance asphérique P(h), ou puissance asphérique, peut s'exprimer sous la forme de l'expression polynomiale suivante :

$$P(h) = \sum_{i=o}^{i=7} a_i \, h^i \qquad \qquad (I)$$

dans laquelle $\underline{h}$ est la hauteur en mm par rapport à l'axe A de la lentille de contact 10 et $a_i$ une succession de coefficients numériques, ici limités à sept.

Cette puissance asphérique P(h) peut également être considérée comme résultant de l'addition à une puissance sphérique de base $P_s$, d'une puissance d'asphérisation $P_a(h)$ :

$$P(h) = P_s + P_a(h)$$

Physiquement, la lentille de contact 10 peut par ailleurs être assimilée à une lame de phase, dont la loi de variation de phase, ou loi de phase, en fonction de la hauteur $\underline{h}$, est donnée par l'expression suivante :

$$\Phi(h) = 2\pi / l \, . \int_{o}^{h} u \, . \, P(u) \, . \, du \qquad \qquad (II)$$

dans laquelle $l$ est la longueur d'onde correspondant à la sensibilité photopique maximale de l'oeil.

Pour une lentille de contact unifocale à surfaces sphériques, cette loi de phase est représentative de la variation d'épaisseur de cette lentille par rapport à une lame à faces parallèles.

Suivant l'invention, pour la fabrication d'une lentille de contact 10 présentant le profil de puissance asphérique recherché qui est celui représenté à la figure 2, on part d'une lentille de contact présentant un autre profil de puissance, et, par un processus de traitement physico-chimique, on assure une modification de ce profil de puissance conduisant à celui recherché.

Par exemple, la lentille de contact de départ, qui n'est pas représentée sur les figures, est une lentille de contact sphérique, de puissance $P_o$.

Si l'on choisit $P_o = P_s$, la puissance d'asphérisation $P_a(h)$ à appliquer s'écrit comme suit :

$$P_a(h) = P(h) - P_o \quad \text{(III)}$$

soit, en terme de phase, :

$$\Phi_a = 2\pi / 1 \cdot \int_0^h u \cdot P_a(u) \cdot du \qquad \text{(III)'}$$

Dans le cas le plus général, le processus de traitement physico-chimique mis en oeuvre peut conduire à la fois à une modulation $D_n$ de l'indice de réfraction et à une modulation $D_e$ de l'épaisseur.

Moyennant une approximation du premier ordre, la modulation de phase $D\Phi$ correspondante peut s'écrire sous la forme de l'expression suivante :

$$D\Phi = 2\pi / l \, [D_n \cdot e + (n - n_o) \, D_e] \quad \text{(IV)}$$

dans laquelle $n$ est l'indice de réfraction du matériau constitutif de la lentille de contact 10 et $n_o$ celui du milieu extérieur.

Il résulte de ce qui précède que les expressions (III) et (IV) doivent être égales.

Autrement dit, il faut avoir l'égalité suivante :

$$D_n(h) \cdot e + (n - n_o) \cdot D_e(h) = \int_0^h u \cdot P_a(u) \cdot du$$

Deux cas de figure peuvent alors se présenter, suivant la nature du processus de traitement physico-chimique.

Dans le premier cas, la "dynamique" de ce processus, c'est-à-dire la modulation maximale d'indice de réfraction pure et/ou la modulation maximale d'épaisseur pure susceptibles d'être obtenues en application de ce processus sont suffisantes pour obtenir directement l'excursion de phase nécessaire.

Dans un deuxième cas, cette "dynamique" n'est pas suffisante pour atteindre cette variation de phase.

Dans un tel cas, une première solution peut consister à modifier la puissance sphérique $P_o$ de la lentille de contact de départ, de façon à minimiser l'excursion de phase nécessaire à l'asphérisation recherchée.

Il suffit pour ce faire de rajouter à la puissance sphérique de base $P_s$ une puissance dynamique $P_d$, ou d'en soustraire cette puissance dynamique $P_d$.

Si, malgré cela, la "dynamique" du processus de traitement physico-chimique mis en oeuvre n'est encore pas suffisante pour atteindre l'excursion de phase nécessaire, une deuxième solution consiste, suivant l'invention, à procéder à une réduction modulo $2\pi$ de la loi de phase.

En effet, ainsi qu'on le sait, la phase est toujours définie à $2\pi$ près.

En procédant à une réduction modulo $2\pi$ de la loi de phase, il est donc toujours possible de maintenir en dessous de $2\pi$ l'excursion de phase à assurer.

Comme indiqué ci-dessus, les processus de traitement physico-chimique mis en oeuvre pour l'asphérisation recherchée peuvent être très divers.

Par exemple, il peut s'agir d'un processus photochimique sur lequel, partant d'une lentille de contact en un matériau polymère solide formant matrice, on imprègne cette matrice au moyen d'une composition liquide photo-polymérisable comportant un monomère et un photoinitiateur de polymérisation, on expose la matrice ainsi imprégnée à une source de rayonnement modulée suivant l'asphérisation recherchée, de manière à polymériser localement la composition liquide qui l'imprègne en fonction de cette asphérisation, puis on élimine de la matrice l'excès non polymérisé de cette composition.

Pour moduler le rayonnement à appliquer à la matrice, on procède à une modulation spatiale du flux d'énergie correspondant.

Suivant l'invention, cette modulation spatiale est assurée par mise en oeuvre d'un masque tournant (12).

Pour une meilleure illustration de l'invention, on en donnera maintenant, à titre indicatif, deux exemples numériques.

Exemple 1.

Il s'agit d'une lentille de contact convergente 10 de puissance asphérique totale P(h) dont la puissance sphérique de base vaut $P_s$ et dont l'addition $A_{dd}$ apportée par l'asphérisation doit valoir 1,5 dioptries.

Par addition $A_{dd}$ on entend ici, de manière conventionnelle, la différence entre la puissance en vision de près $P_{VP}$ et la puissance en vision de loin $P_{VL}$, figure 2, étant entendu que les zones correspondantes de vision de près VP et de vision de loin VL de la lentille de contact 10 ont une certaine extension géométrique et que leur puissance est la puissance moyenne pour l'ensemble d'une telle zone.

Comme précédemment :

$$P(h) = P_s + P_a(h)$$

et

$$P_a(h) = \sum_{i=0}^{i=7} a_i \, h^i$$

Dans l'exemple numérique retenu, les coefficients $a_i$ ont la valeur suivante :
a0 = 1.8430572
a1 = 0.030438234
a2 = 0.92669499
a3 = 0.52752879
a4 = 1.1186199
a5 = 0.79106624
a6 = 0.30761219
a7 = 0.04890526

Par ailleurs, et par simple commodité, $P_s$ est choisie nulle :

$$P(s) = o$$

On supposera tout d'abord que la lentille de contact de départ présente elle aussi une puissance sphérique nulle.

Par exemple, il s'agit d'une lentille à faces parallèles.

Quoi qu'il en soit :

$$P_o = o$$

Sur le diagramme de la figure 2, sont portées en abscisses, la hauteur $\underline{h}$, en mm, et, en ordonnées, la puissance d'asphérisation $P_a$, en dioptries, à appliquer à la zone optique utile de cette lentille de contact de départ pour avoir le profil de puissance asphérique recherché pour la lentille de contact 10.

En hachuré, ont été reportées, sur ce diagramme, les zones de vison de près VP et de vision de loin VL de cette lentille de contact 10.

Dans l'exemple donné, la zone de vision de près VP appartient à la zone centrale de la lentille de contact 10, et la zone de vision de loin VL à sa zone périphérique.

Mais il va de soi qu'une disposition inverse peut être obtenue.

Le diagramme de la figure 3, donne l'allure de la loi de phase correspondante.

Par commodité, sur ce diagramme, la phase $\Phi$ est rapportée à $2\pi$.

Il ressort de ce diagramme que le rapport correspondant s'étend entre zéro et 2,5.

L'excursion de phase à couvrir est donc de l'ordre de $5\pi$.

Si le processus de traitement physico-chimique mis en oeuvre entraîne une modulation d'indice pure, celle-ci devra atteindre une valeur de $7.10^{-3}$ pour une lentille de contact de départ présentant une épaisseur de 200 μm.

Si ce processus de traitement physico-chimique entraîne une modulation d'épaisseur pure, celle-ci devra atteindre 3,6 μm si l'indice de réfraction du matériau constitutif de la lentille de contact de départ est de 1,38.

5

Par exemple, le processus de traitement physico-chimique mis en oeuvre est un processus photochimique du type de celui succinctement exposé ci-dessus.

Ce processus photochimique est plus complètement décrit dans la demande de brevet français No 89 06323 déposée le 12 Mai 1989 et publiée sous le No 2.646.930.

La figure 4 donne l'allure d'un masque tournant (12) propre à la modulation spatiale du flux d'énergie d'irradiation correspondant.

Sur cette figure 4, les parties opaques du masque (12) ont été hachurées, et ses parties transparentes laissées en blanc.

On supposera maintenant que la lentille de contact de départ présente une puissance sphérique $P_o$ non nulle.

Par exemple :

$$P_o = 0,6 \text{ dioptrie}$$

Dans ce cas :

$$P_o = P_s + P_d$$

avec

$$P_d = 0,6 \text{ dioptrie}$$

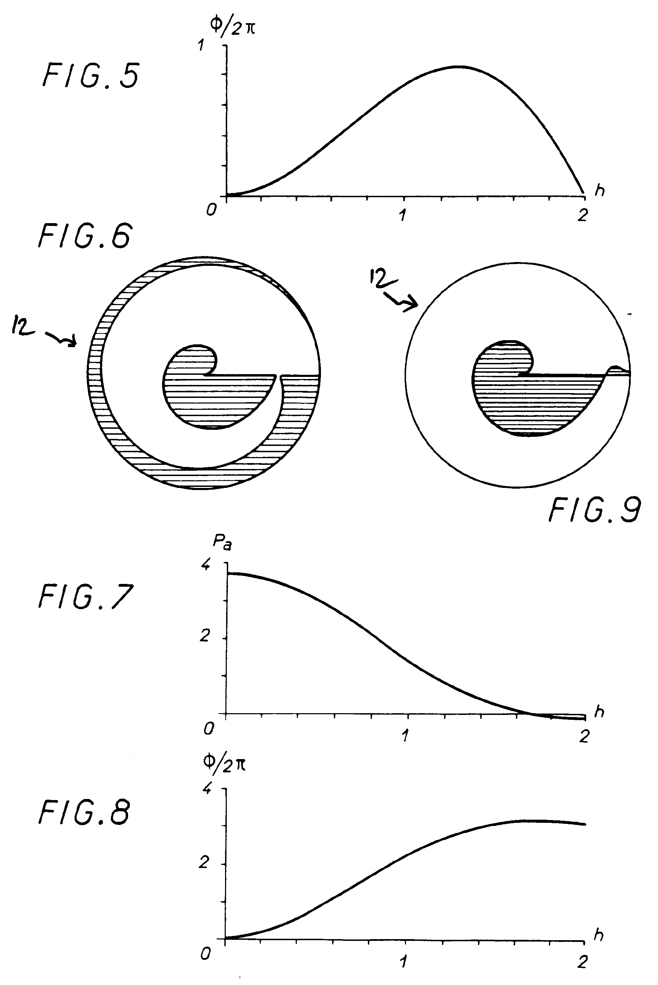

La loi de phase se trouve dès lors réduite, suivant le diagramme de la figure 5.

Dans le cas d'une modulation d'indice pure, celle-ci se réduit à $2,8.10^{-3}$.

Dans le cas d'une modulation d'épaisseur pure, celle-ci se réduit à 1,4 μm.

La figure 6 donne l'allure du masque tournant (12) correspondant.

Exemple 2.

L'addition $A_{dd}$ à rapporter par asphérisation, dans les mêmes conditions que ci-dessus, vaut 2,5 dioptries.

Les coefficients $a_i$ sont alors les suivants :

a0 = 3.7110522
a1 = 0.040382098
a2 = 3.1321414
a3 = 0.034659945
a4 = 0.99060474
a5 = 0.098949193
a6 = 0.10272511
a7 = 0.01788491

Si, comme précédemment, $P_s$ et $P_o$ sont égales à zéro, la loi de variation de la puissance d'asphérisation $P_a$ est celle donnée par le diagramme de la figure 7, et la loi de phase est celle donnée par le diagramme de la figure 8.

L'excursion de phase à couvrir est de l'ordre de $6\pi$.

Dans le cas d'une modulation d'indice pure, celle-ci doit atteindre une valeur de $8,3.10^{-3}$ pour une lentille de contact de départ d'épaisseur de 200 μm.

Dans le cas d'une modulation d'épaisseur pure, celle-ci doit atteindre 4,3 μm pour une lentille de contact de départ dont le matériau constitutif a un indice de réfraction égal à 1,38.

Le masque tournant (12) à mettre en oeuvre est celui représenté à la figure 9.

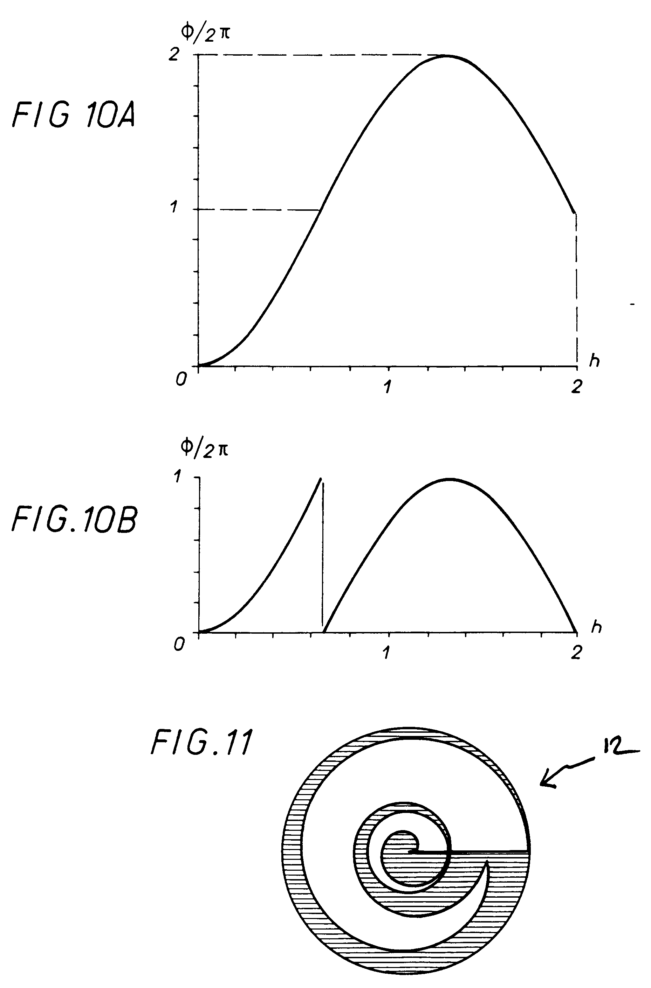

Mais, si la lentille de contact de départ présente une puissance positive $P_o$ non nulle, et par exemple une puissance positive égale à 0,6 dioptrie, la loi de phase devient celle donnée par le diagramme de la figure 10A.

Ainsi que l'illustre le diagramme de la figure 10B, il est possible de procéder à une réduction modulo $2\pi$ de cette loi de phase.

Dans le cas d'une modulation d'indice pure, celle-ci se réduit dès lors à $2,8.10^{-3}$.

Dans le cas d'une modulation d'épaisseur pure, celle-ci se réduit à 1,4 μm.

La figure 11 donne l'allure du masque tournant (12) correspondant.

Bien entendu, les exemples numériques ainsi donnés ne doivent en rien être considérés comme restrictifs de l'invention.

Au contraire, l'invention s'étend aussi bien à toute variante d'exécution et de mise en oeuvre.

L'invention est en particulier applicable à l'obtention de lentilles de contact unifocales mais également de lentilles de contact sphéro-toriques pour la correction de l'astigmatisme.

Partant d'une lentille de contact de puissance constante P, on peut réaliser au moyen du procédé suivant l'invention une lentille de contact de puissance constante supérieure $(P + \Delta P)$.

Exemples :

```
Soit Δ P = 0,5 D
La phase maximale pour h = 2 mm est       Φ    = 4π
Pour une modulation d'épaisseur pure      De   = 2,9μm
Pour une modulation d'indice pure         Dn   = 5,5.10⁻³
```

```
Soit Δ P = 0,25 D
La phase maximale pour h = 2 mm est       Φ    = 2π
Pour une modulation d'épaisseur pure      De   = 1,45μm
Pour une modulation d'indice pure         Dn   = 2,75.10⁻³
```

On peut également au moyen du procédé suivant l'invention réaliser des lentilles artificielles permettant de corriger simultanément l'astigmatisme et la presbytie. Dans ce cas on part d'une lentille artificielle sphéro-torique, c'est-à-dire d'une lentille dont l'une des surfaces est sphérique tandis que l'autre est torique.

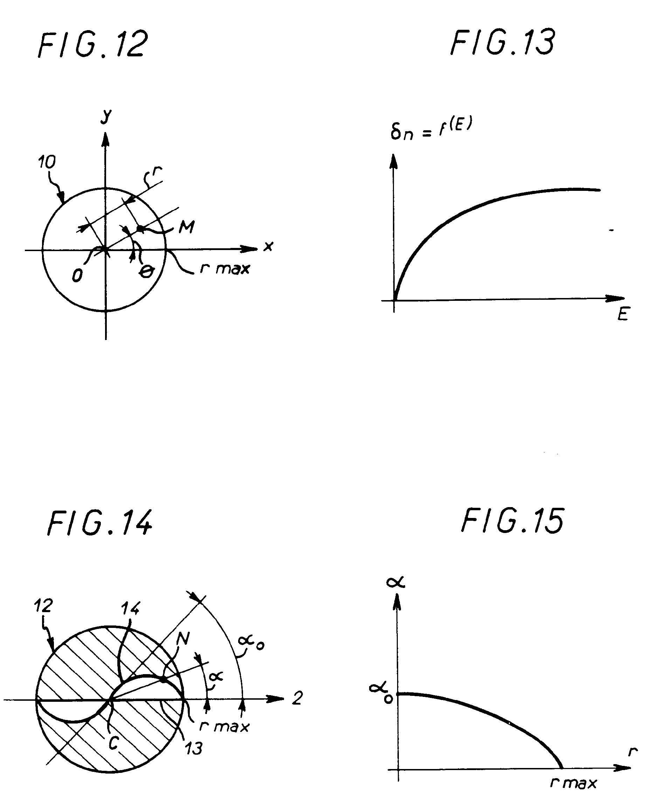

On décrira maintenant, en référence aux figures 12 à 15 un développement de l'invention propre à l'obtention d'un profil de puissance astigmate, ou, autrement dit, à la réalisation de lentilles optiques artificielles dont les profils de puissance suivant deux axes orthogonaux sont différents.

En coordonnées polaires, la loi de phase de la lame de phase à laquelle peut être assimilée une lentille de contact torique, c'est-à-dire une lentille de contact propre à la correction de l'astigmatisme, est, à la manière de la formule (II) ci-dessus, du type de la suivante :

$$\Phi\ (r,\ \Theta)\ =\ \frac{2\pi}{\lambda}\cdot\frac{r^2}{2}\ (\cos^2\Theta\ .\ Px\ +\ \sin^2\Theta\ Py)\quad (II')$$

dans laquelle :

$\lambda$ est la longueur d'onde correspondant à la sensibilité photopique maximale de l'oeil,

Px et Py sont les puissances sur deux axes orthogonaux Ox, Oy,

r la distance à l'origine O d'un point M quelconque de la lentille de contact 10 concernée et $\Theta$ l'angle que fait avec l'axe Ox le rayon correspondant, figure 1.

Pour une lentille de contact, la zone optique utile doit avoir au moins un diamètre de 6 mm.
Soit, donc :

$$r\ max\ =\ 3.$$

Par mesure de simplicité, et à titre d'exemple, on supposera ci-après que l'astigmatisme à obtenir pour la lentille de contact 10 recherchée est au plus égal à deux dioptries.
Soit, donc :

$$\delta Pmax\ =\ |\ Px\ -\ Py\ |\ max\ =\ 2d$$

Il en résulte :

$$\delta\Phi max\ =\ 33\pi$$

On supposera également ci-après, à titre d'exemple, que, comme dans ce qui précède, la lentille de contact de départ est une lentille de contact sphérique, mais que, ici, cette lentille de contact sphérique, non représentée, est d'épaisseur constante.

On supposera, enfin, que, suivant des modalités de mise en oeuvre décrites ci-dessus, on applique à cette lentille de contact de départ un processus photochimique, en l'espèce un processus de photopolymérisation, permettant d'obtenir en son sein une variation locale d'indice de réfraction de nature à modifier son profil de puissance, initialement nul, pour conduire au profil de puissance astigmate recherché, en mettant en oeuvre, pour la modulation spatiale du flux d'énergie correspondant, un masque tournant.

En pratique, et suivant le diagramme de la figure 2, la variation locale d'indice de réfraction $\delta n$ est fonction de l'énergie E reçue pendant ce processus photochimique.
Soit donc :

$$\delta n\ =\ f\,(E)\quad (V)$$

Mais, eu égard à la relation (II') précédente, cette variation locale d'indice de réfraction $\delta n$ peut, également, en coordonnées polaires, s'écrire sous la forme de la relation suivante :

$$\delta n\ (r,\ \Theta)\ =\ \frac{1}{K}\frac{r^2}{2}\ (\cos^2\Theta\ .\ \delta P\ +\ Py)\quad (II'')$$

7

dans laquelle K est une constante.

Il en résulte la relation suivante :

$$E(r, \Theta) = f^{-1}\left[\frac{r^2}{2K}(Cos^2\Theta . \delta P + Py)\right] \quad (VI)$$

On supposera maintenant que, tel que représenté à la figure 14, les parties transparentes du masque tournant 12 mis en oeuvre sont chacune délimitées, d'une part, par une droite 13, prise ci-après comme axe de référence pour la distance r, et, d'autre part, par une courbe 14 qui, passant par le centre C, recoupe au point r max la courbe 14 précédente.

Soit r et $\alpha$ (r) les coordonnées polaires d'un point N de la courbe 14, et $\alpha$ (o) l'angle de sa tangente à l'origine C.

Le diagramme de la figure 15 donne l'allure de la courbe représentative de l'angle $\alpha$.

Soit, enfin, v ($\Theta$) la vitesse de rotation du masque tournant 12.

L'énergie que laisse passer ce masque tournant 12 au cours du processus photochimique appliqué à la lentille de contact de base peut s'écrire comme suit :

$$E(r, \Theta_o) = E_o . \int_{\Theta_o - \frac{\alpha(r)}{2}}^{\Theta_o + \frac{\alpha(r)}{2}} \frac{d\Theta}{v(\Theta)} \quad (VII)$$

Si la vitesse de rotation v($\Theta$) est constante, et égale par exemple à $v_o$, et si la loi $\underline{f}$ est linéaire, on obtient une lentille de contact sphérique de puissance $P_o$.

Les relations correspondantes donnent $E_o$ et $\Theta_o$.

S'agissant, suivant l'invention, de l'obtention d'une lentille de contact torique, c'est-à-dire d'une lentille de contact présentant un profil de puissance astigmate, on fait varier la vitesse de rotation $\underline{v}$ suivant la position angulaire relative du masque tournant 12 par rapport à la lentille de contact 10 traitée, en supposant cette position angulaire mesurée par l'angle $\Theta$.

Il résulte alors des relations (VI) et (VII) précédentes la nouvelle relation suivante :

$$f^{-1}\left[\frac{r^2}{2K}(cos^2\Theta_o . \delta P + Py\right] = E_o . \int_{\Theta_o - \frac{\alpha(r)}{2}}^{\Theta_o + \frac{\alpha(r)}{2}} \frac{d\Theta}{v(\Theta)}$$

soit :

$$r^2(Cos2\Theta_o . \delta P + Px + Py) = K' . \int_{\Theta_o - \frac{\alpha(r)}{2}}^{\Theta_o + \frac{\alpha(r)}{2}} \frac{d\Theta}{v\Theta} \quad (VIII)$$

dans laquelle K' est une constante.

La relation (VIII) ainsi obtenue est une équation intégrale permettant de déterminer la vitesse v($\Theta$) recherchée.

Le calcul montre que, en première approximation, c'est-à-dire en négligeant le terme en $\alpha^2$, les aberrations auxquelles conduise cette approximation se révélant en pratique parfaitement acceptables, il existe, pour cette équation intégrale, une solution de la forme :

$$\begin{cases} v\ (\theta)\ =\ \dfrac{v_o}{(Px\ +\ Py)\ +\ (Px\ -\ Py)\ \cos 2\theta} \\[2em] \alpha\ (r)\ =\ \alpha_o\ .\ r^2 \end{cases}$$

dans laquelle, comme succinctement indiqué ci-dessus, $\Theta$ est l'angle relatif entre le masque tournant 12 et la lentille optique artificielle en cours de traitement, en l'espèce, la lentille de contact 10.

Il est ainsi possible de déterminer, de manière très simple, pour une allure de courbe donnée pour la courbe 14 délimitant les parties transparentes du masque tournant 12 mis en oeuvre, en pratique une conique, la vitesse de rotation $v(\Theta)$ à appliquer à ce masque tournant 12 pour obtenir avec une approximation largement suffisante, le profil de puissance astigmate recherché pour la lentille de contact 10 traitée.

Pour une meilleure illustration de l'invention, on en donnera ci-après plusieurs exemples numériques.

I. astigmastisme : 2 d

      puissance sphérique : - 4 d

       a) lentille de base : - 1,75 d

           Px = - 0,25 d

           Py = - 2,25 d

           $\alpha\ (r) = \alpha_o\ \dfrac{r^2}{rmax^2}\ \alpha_o = 10°$

           $\delta n = 5.10^{-2}$         $\delta\Phi max = 37\pi$

       b) lentille de base : - 4,25 d

           Px = + 2,25 d

           Py = + 0,25 d

           $\alpha\ (r) = \alpha_o\ (1 - \dfrac{r^2}{rmax^2})\ \alpha_o = 10°$

           $\delta n = 5.10^{-2}$         $\delta\Phi max = 37\pi$

II. astigmatisme : 1 d

      puissance sphérique : - 2 d

       a) lentille de base : - 0,75 d

           Px = - 0,25 d

           Py = - 1,25 d

           $\alpha\ (r) = \alpha_o\ \dfrac{r^2}{rmax^2}\ \alpha_o = 10°$

           $\delta n = 2,8\ .\ 10^{-2}$         $\delta\Phi max = 21\pi$

       b) lentille de base : - 2,25 d

           Px = + 1,25 d

           Py = + 0,25 d

           $\alpha\ (r) = \alpha_o\ (1 - \dfrac{r^2}{rmax^2})\ \alpha_o = 10°$

           $\delta n = 2,8\ .\ 10^{-2}$         $\delta\Phi max = 21\pi$

Bien entendu la présente invention ne se limite pas à ces exemples numériques mais s'étend à toute variante de mise en oeuvre.

Son domaine d'application n'est en outre pas limité à celui des seules lentilles de contact, mais s'étend au contraire tout aussi bien à l'ensemble des lentilles optiques artificielles, et, donc, aussi bien à celui des lentilles ophtalmiques qu'à celui des implants oculaires.

Dans tous les cas, et, suivant l'invention, ces lentilles optiques artificielles présentent des caractéristiques optiques, le cas échéant des caractéristiques optiques astigmates, résultant d'une modification par processus physico-chimique de caractéristiques optiques initialement différentes.

Enfin, si les exemples de réalisation précédemment donnés concernent plus particulièrement des lentilles optiques artificielles de révolution, il va de soi que la présente invention s'applique tout aussi bien à la réalisation de n'importe quel autre type de lentilles optiques artificielles.

**Revendications**

1. Procédé pour la fabrication d'une lentille optique artificielle présentant un quelconque profil de puissance donné, du genre suivant lequel, partant d'une lentille optique artificielle présentant un autre profil de puissance, on assure par un processus de traitement physico-chimique, une modification de ce profil de puissance conduisant au profil de puissance recherché, avec, pour la modulation spatiale du flux d'énergie à contrôler, l'intervention d'un masque, caractérisé en ce que on met en oeuvre, pour la modulation spatiale du flux d'énergie, un masque tournant (12).

2.  Procédé suivant la revendication 1, caractérisé en ce que on part d'une lentille optique artificielle sphérique ou sphéro-torique.

3.  Procédé suivant l'une quelconque des revendications 1, 2, caractérisé en ce que, la modification à appliquer étant exprimée en loi de phase, on assure une réduction de cette loi de phase de nature à maintenir en dessous de $2\pi$ l'excursion de phase nécessaire.

4.  Procédé suivant la revendication 3, caractérisé en ce que, la modification à appliquer étant exprimée en loi de phase, on assure, si nécessaire, une réduction modulo $\pi$ de cette loi de phase.

5.  Procédé suivant l'une quelconque des revendications 1 à 4, caractérisé en ce que le processus de traitement physico-chimique mis en oeuvre est choisi de manière à permettre une modulation de l'une au moins des caractéristiques suivantes : indice de réfraction, épaisseur.

6.  Procédé suivant l'une quelconque des revendications 1 à 5, caractérisé en ce que le processus de traitement physico-chimique mis en oeuvre est un processus photochimique.

7.  Procédé suivant l'une quelconque des revendications 1 à 6, caractérisé en ce que, pour l'obtention d'un profil de puissance astigmate, on fait varier la vitesse de rotation du masque tournant (12) suivant la position angulaire relative ($\Theta$) de ce masque tournant (12) par rapport à la lentille optique artificielle (10) traitée.

8.  Procédé suivant la revendication 7, caractérisé en ce qu'on choisit pour loi de vitesse (v) du masque tournant (12) une loi de vitesse de la forme :

$$v\ (\Theta)\ =\ \frac{v_o}{(Px\ +\ Py)\ +\ (Px\ -\ Py)\ \cos 2\Theta}$$

dans laquelle
    v est la vitesse
    $\Theta$ l'angle relatif entre le masque tournant (12) et la lentille optique artificielle (10) en cours de traitement
    Px et Py les puissances à obtenir suivant deux axes orthogonaux (Ox, Oy).

9.  Procédé suivant l'une quelconque des revendications 7, 8, caractérisé en ce qu'on délimite les parties transparentes du masque tournant (12) d'une part par une droite (13), et d'autre part par une courbe (14) qui, passant par le centre (C) de ce masque tournant, recoupe ladite droite (13).

10. Procédé suivant la revendication 9, caractérisé en ce que ladite courbe (14) est une conique.


**Patentansprüche**

1.  Verfahren zur Herstellung einer künstlichen optischen Linse, die irgendein Stärkeprofil zeigt, derart, daß man ausgehend von einer künstlichen optischen Linse, die ein anderes Stärkeprofil zeigt, durch ein physikalisch-chemisches Behandlungsverfahren eine Veränderung dieses Stärkeprofils gewährleistet, die zu dem gesuchten Stärkeprofil führt, mit dem Eingriff einer Maske für die räumliche Modulation des zu steuernden Energiestromes, dadurch gekennzeichnet, daß man für die räumliche Modulation des Energiestromes eine sich drehende Maske (12) einsetzt.

2.  Verfahren gemäß Anspruch 1, dadurch gekennzeichnet, daß man von einer sphärischen oder sphärisch-torischen künstlichen optischen Linse ausgeht.

3. Verfahren gemäß irgendeinem der Ansprüche 1, 2, dadurch gekennzeichnet, daß man, da die anzuwendende Veränderung im Phasengesetz ausgedrückt ist, eine Reduktion dieses natürlichen Phasengesetzes gewährleistet, wobei die notwendige Phasenabweichung unter 2 π zu halten ist.

4. Verfahren gemäß Anspruch 3, dadurch gekennzeichnet, daß man, da die anzuwendende Veränderung im Phasengesetz ausgedrückt ist, falls notwendig, eine Reduktion modulo π dieses Phasengesetzes gewährleistet.

5. Verfahren gemäß irgendeinem der Ansprüche 1 bis 4, dadurch gekennzeichnet, daß das eingesetzte physikalisch-chemische Behandlungsverfahren so gewählt ist, daß es eine Modulation von zumindest einer der folgenden Eigenschaften erlaubt Brechungsindex, Dicke.

6. Verfahren gemäß irgendeinem der Ansprüche 1 bis 5, dadurch gekennzeichnet, daß das eingesetzte physikalisch-chemische Behandlungsverfahren ein photochemisches Verfahren ist.

7. Verfahren gemäß irgendeinem der Ansprüche 1 bis 6, dadurch gekennzeichnet, daß man zum Erhalt eines astigmatischen Stärkeprofils die Drehgeschwindigkeit der sich drehenden Maske (12) gemäß der relativen Winkelposition (Θ) dieser sich drehenden Maske (12) bezüglich der behandelten künstlichen optischen Linse (10) ändert.

8. Verfahren gemäß Anspruch 7, dadurch gekennzeichnet, daß man als Geschwindigkeitsgesetz (v) der sich drehenden Maske (12) ein Geschwindigkeitsgesetz der Form wählt :

$$v(\Theta) = \frac{v_O}{(Px + Py) + (Px - Py) \quad \cos 2\Theta}$$

bei der v die Geschwindigkeit ist

Θ der relative Winkel zwischen der sich drehenden Maske (12) und der in der Behandlung befindlichen künstlichen optischen Linse (10)

Px und Py die Stärken, die gemäß zwei rechtwinkligen Achsen (Ox, Oy) erhalten werden.

9. Verfahren gemäß irgendeinem der Ansprüche 7, 8, dadurch gekennzeichnet, daß man die transparenten Abschnitte der sich drehenden Maske (12) einerseits durch eine Gerade (13) und andererseits durch eine Kurve (14) begrenzt, die durch den Mittelpunkt (C) dieser sich drehenden Maske gehend die Gerade (13) schneidet.

10. Verfahren gemäß Anspruch 9, dadurch gekennzeichnet, daß die Kurve (14) eine konische Kurve ist.

## Claims

1. A method of manufacturing an artificial optical lens which has any given power profile. of the type in which an initial artificial optical lens is taken which has a different power profile and a physical-chemical treatment method is used to modify that power profile to produce the desired power profile, a mask coming into play to give spatial modulation of the energy flux to be controlled, characterised in that a rotating mask (12) is used for the spatial modulation of the energy flux.

2. A method according to Claim 1, characterised in that the initial lens is a spherical or spherical-toroidal artificial optical lens.

3. A method according to any one of Claims 1, 2, characterised in that the modification which is to be made is expressed as a phase law, and that phase law is reduced in such a way that the necessary phase excursion is kept below $2\pi$.

11

4. A method according to Claim 3, characterised in that the modification to be made is expressed as a phase law, and, if necessary, said phase law is reduced modulo $\pi$.

5. A method according to any one of Claims 1 to 4, characterised in that the physical-chemical treatment method used is selected in such a way as to permit modulation of at least one of the following characteristics: refraction index, thickness.

6. A method according to any one of Claims 1 to 5, characterised in that the physical-chemical treatment method used is a photochemical process.

7. A method according to any one of Claims 1 to 6, characterised in that to produce an astigmatic power profile the speed of rotation of the rotating mask (12) is varied in accordance with the angular position ($\Theta$) of the rotating mask (12) relative to the artificial optical lens (10) treated.

8. A method according to Claim 7, characterised in that the speed law (v) of the rotating mask (12) is selected as a speed law of the form: wherein:

$$v\,(\theta)\;=\;\frac{V_o}{(Px\,+\,Py)\,+\,(Px\,-\,Py)\;\;\cos 2\theta}$$

wherein:
v is the speed
$\Theta$ is the relative angle between the rotating mask (12) and the artificial optical lens (10) during the treatment
Px and Py are the powers to be obtained along two orthogonal axes (Ox, Oy).

9. A method according to any one of Claims 7, 8, characterised in that the transparent parts of the rotating mask (12) are delimited by a straight line (13), on the one hand, and by a curve (14), on the other hand, which passes through the centre (C) of the rotating mask and intersects said straight line (13).

10. A method according to Claim 9, characterised in that said curve (14) is a conical section.

EP 0 439 394 B1

FIG.1

FIG.2

FIG.3

FIG.4

13

EP 0 439 394 B1

FIG.5

$\phi/2\pi$

FIG.6

FIG.7

$P_a$

FIG.8

$\phi/2\pi$

FIG.9

14

FIG 10A

FIG.10B

FIG.11

15

## FIG.12

## FIG.13

$$\delta_n = f^{(E)}$$

## FIG.14

## FIG.15